# EUROPÄISCHE PATENTSCHRIFT

(11) **EP 1 156 994 B1**
(45) Veröffentlichungstag und Bekanntmachung des Hinweises auf die Patenterteilung: **14.04.2004**
(21) Anmeldenummer: 00910696.4
(22) Anmeldetag: 24.02.2000
(51) Int. Cl.: C07C 41/03, C07C 67/26, C07C 41/34, C07C 43/11

(54) **VERFAHREN ZUR HERSTELLUNG ALKOXYLIERTER NICHTIONISCHER TENSIDE**
METHOD OF PREPARING ALKOXYLATED NON-IONIC SURFACE-ACTIVE AGENTS
PROCEDE DE PREPARATION DE TENSIOACTIFS NON IONIQUES ALCOXYLES

(30) Priorität: 03.03.1999 DE 19909272
(43) Veröffentlichungstag der Anmeldung: 28.11.2001
(73) Patentinhaber: Cognis Deutschland GmbH & Co. KG, 40589 Düsseldorf (DE)
(72) Erfinder: BEHLER, Ansgar, D-46240 Bottrop (DE); SCHARES, Horst-Dieter, D-40699 Erkrath (DE); FOLGE, Almud, D-40764 Langenfeld (DE)
(86) Internationale Anmeldenummer: PCT/EP2000/001502
(87) Internationale Veröffentlichungsnummer: WO 2000/051955

(56) Entgegenhaltungen:
- WO-A-92/12950
- WO-A-92/12951
- DE-A- 3 843 713
- DE-A- 4 446 064
- DE-C- 19 611 999

## Beschreibung

Die Erfindung betrifft ein Verfahren zur Herstellung alkoxylierter nichtionischer Tenside, bei dem man Verbindungen mit aktiven Wasserstoffatomen oder Carbonsäureester mit Alkylenoxiden in Gegenwart von ggf. modifiziertem Hydrotalcit als Katalysator sowie ggf. weiteren ausgewählten Co-Katalysatoren umsetzt und anschließend mit Säuren nachbehandelt.

Eine wichtige Gruppe der nichtionischen Tenside stellen Anlagerungsprodukte von Alkylenoxiden, insbesondere Ethylenoxid und/oder Propylenoxid an Verbindungen mit aktivem Wasserstoff dar, die üblicherweise mittels homogener Katalyse in Gegenwart von Alkalimetallhydroxiden oder Alkalimetallalkoholaten hergestellt werden. Nach dem homogen katalysierten Verfahren werden Produkte mit einer breiten Homologenverteilung erhalten. Produkte mit eingeschränkter Homologenverteilung können erhalten werden, wenn die Umsetzung in Gegenwart von ggf. modifizierten Hydrotalciten, beispielsweise gemäß der deutschen Offenlegungsschrift **DE-A-3833076 und DE-C-196 11 999** erfolgt. Auch die Alkoxylierung von Carbonsäureestern erfolgt mit besseren Erfolgen in Gegenwart von Hydrotalciten, wobei die Alkylenoxide in die Carbonylesterbindung eingelagert (Insertion) werden, beispielsweise gemäß den beiden Patentschriften **EP-B1- 0 339 425** und **EP-B1- 0 523 089.**

Nach Durchführung der eigentlichen Alkoxylierung unter Einsatz von ggf. modifiziertem Hydrotalcit bereitet jedoch die Abtrennung des Katalysators vom Reaktionsprodukt technische Schwierigkeiten, da der ggf. modifizierte Hydrotalcit in der Regel so feinteilig ist, daß die Filtration nur über spezielle Filterkerzen gelingt. Ein Verbleiben des Katalysators im Reaktionsendprodukt ist indes auch nicht möglich, da es sonst zu Austrübungen und zu Sedimentationen kommen kann. Aus der **WO 92/12950** ist ein Verfahren zur Herstellung von Polyalkylenglykolestern bekannt, wobei die Reaktionsprodukte mit einem nicht-polymeren Fällungsmittel versetzt werden.
Auch in der **WO 92/12951** wird bei der Herstellung von Fettalkoholpolyglycolethem ein Koagulierungsmittel zugesetzt.
Die **DE-A1-44 46 064** offenbart ein Verfahren zur Herstellung von Polyoxyalkylenalkylether-Fettsäuren, wobei der Katalysator in üblicher Weise durch Filtration entfernt wird.

Die Aufgabe der vorliegenden Erfindung hat somit darin bestanden, ein Verfahren zur Herstellung von alkoxylierten nichtionischen Tensiden zur Verfügung zu stellen, welches die Nachteile der aufwendigen Filtration bzw. der Austrübungen und Sedimentationen des Reaktionsendproduktes nicht mehr aufweist.

Überraschenderweise konnte die Aufgabe der vorliegenden Erfindung gelöst werden, wenn man nach der Alkoxylierung den Katalysator Hydrotalcit sowie ggf. ebenfalls vorhandene Co-Katalysatoren nicht abfiltriert, sondern durch Zugabe von Säuren zersetzt. Die Erfindung schließt die Erkenntnis ein, daß eine derartige nachgeschaltete Säurebehandlung den Hydrotalcit sowie die ggf. vorhandenen Co-Katalysatoren in solche Produkte zersetzt, die in der Reaktionsmischung verbleiben können, ohne daß es später zu Austrübungen oder Sedimentationen kommt.

Ein Gegenstand der vorliegenden Erfindung betrifft daher ein Verfahren zur Herstellung alkoxylierter nichtionischer Tenside durch Umsetzung von Carbonsäuren mit 6 bis 22 Kohlenstoffatomen oder Carbonsäureestern mit Alkylenoxiden in Gegenwart von ggf. modifiziertem Hydrotalcit als Katalysator und ggf. von Co-Katalysatoren, dadurch gekennzeichnet, dass nach der Alkoxylierung Säuren in Form wässriger Lösungen im Verhältnis zu Hydrotalcit und ggf. Co-Katalysatoren in molaren Mengen von 1 : 1 bis 10 : 1 bei Temperaturen zwischen 70 und 95 °C zugegeben werden.

Als **Verbindungen** mit aktiven Wasserstoffatomen kommen die folgenden Stoffklassen in Betracht:
Carbonsäuren mit 6 bis 22 Kohlenstoffatomen (sogenannte Fettsäuren) und Hydroxyfettsäuren, wie z.B. Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Ricinolsäure, Elaeostearinsäure, Arachinsäure, Gadoleinsäure, Behensäure und Erucasäure sowie deren technische Gemische, wie sie beispielsweise bei der Druckspaltung von natürlichen Fetten und Ölen anfallen. Bevorzugt sind Fettsäuren mit 12 bis 18 Kohlenstoffatomen, beispielsweise technische Kokos- bzw. Talgfettsäuren.

In einer weiteren Ausführungsform des erfindungsgemäßen Verfahrens werden als Ausgangsstoffe Carbonsäureester eingesetzt. Auch hier können grundsätzlich zwei Typen unterschieden werden:
b1) Carbonsäureniedrigalkylester der Formel **(I),**

   **R**^{**1**}**CO-OR**^{**2**} **(I)**

   in der R¹CO für einen aliphatischen Acylrest mit 6 bis 22 Kohlenstoffatomen und R² für einen linearen oder verzweigten Alkylrest mit 1 bis 4 Kohlenstoffatomen steht. Typische Beispiele sind Ester der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Ricinolsäure, Elaeostearinsäure, Arachinsäure, Gadoleinsäure, Behensäure und Erucasäure sowie deren technische Gemische mit Methanol, Ethanol, Propanol oder Butanol. Vorzugsweise werden Methylester von Fettsäuren mit 12 bis 18 Kohlenstoffatomen und insbesondere technische Kokos- bzw. Talgfettsäuremethylester eingesetzt.
b2) Carbonsäureglycerinester der Formel **(II)**, 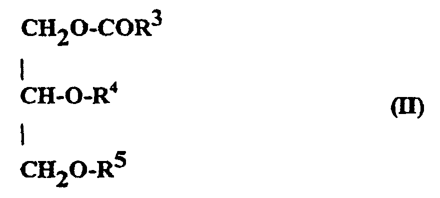 in der R³CO für einen aliphatischen Acylrest mit 6 bis 22 Kohlenstoffatomen und R⁴ und R⁵ unabhängig voneinander für Wasserstoff oder ebenfalls einen aliphatischen Acylrest mit 6 bis 22 Kohlenstoffatomen stehen.

Typische Beispiele hierfür sind synthetische, vorzugsweise jedoch natürliche Triglyceride, wie Palmöl, Palmkernöl, Kokosöl, Rapsöl, Olivenöl, Sonnenblumenöl, Baumwollsaatöl, Erdnußöl, Leinöl, Lardöl, Rindertalg und Schweineschmalz. Vorzugsweise wird Ricinusöl bzw. gehärtetes Ricinusöl eingesetzt.

In einer bevorzugten Ausführungsform werden anstelle der Vollester Fettsäurepartialglyceride, insbesondere Monoglyceride von Fettsäuren mit 12 bis 18 Kohlenstoffatomen eingesetzt. Besonders bevorzugt sind hier technische Kokosfettsäuremonoglyceride.
Innerhalb der Gruppe der Carbonsäureester werden Carbonsäureniedrigalkylester, insbesondere die Methylester der Carbonsäuren mit 6 bis 22 Kohlenstoffatomen bevorzugt.

Nach dem erfindungsgemäßen Verfahren werden als **Alkoxylierungskatalysatoren** ggf. modifizierte Hydrotalcite alleine oder in Mischung mit ausgewählten Co-Katalysatoren eingesetzt. Einer Ausführungsform der vorliegenden Erfindung entsprechend wird ausschließlich ggf. modifizierter Hydrotalcit als Katalysator eingesetzt. Als modifizierte Hydrotalcite werden calcinierte oder hydrophobierte Hydrotalcite, wie sie beispielsweise aus den deutschen Patentanmeldungen **DE-A1 38 43 713** und **DE-A1 40 10 606** (Henkel) bekannt sind, eingesetzt. Besonders bevorzugt werden die calcinierten Hydrotalcite.

Einer weiteren Ausführungsform der vorliegenden Erfindung entsprechend werden als Katalysatoren ggf. modifizierte Hydrotalcite und ausgewählte Co-Katalysatoren zusammen als Alkoxylierungskatalysatoren eingesetzt. Als Co-Katalysatoren eignen sich Verbindungen aus der Gruppe, die gebildet wird von Hydroxiden, Oxiden und/oder Alkoxiden von Alkaliund/oder Erdalkalimetallen sowie von Alkali- und/oder Erdalkalisalzen, Zinnsalzen und von Mischmetalloxiden.

Als Hydroxide von Alkali- und/oder Erdalkalihydroxiden eignen sich insbesondere Lithiumhydroxid und/oder Magnesiumhydroxid.

Innerhalb der Gruppe der Oxide von Alkali- und/oder Erdalkalioxiden werden die Oxide des Magnesiums bevorzugt.

Bevorzugte Alkoxide der Alkali- und/oder Erdalkalimetalle sind solche, die sich von kurzkettigen Alkoholen, beispielsweise von solchen mit 1 bis 8 Kohlenstoffatomen und insbesondere von Methanol, Ethanol und/oder 2-Ethylhexanol ableiten. Hierbei sind die Magnesium- und/oder Bariumverbindungen besonders bevorzugt.

Innerhalb der Gruppe der Alkali- und/oder Erdalkalisalze sind die Magnesium- und Bariumsalze, beispielsweise die Carbonate wie Magnesiumcarbonat, oder die Acetate, beispielsweise Magnesiumacetat von besonderer Bedeutung.

Als Mischmetalloxide werden solche Oxidverbindungen bezeichnet, die mindestens zwei verschiedene Metalle enthalten. Bevorzugt ist eines der Metalle Magnesium. Das andere Metall kann Aluminium, Gallium, Zirkon, Indium, Thallium, Kobalt, Scandium, Lanthan und/oder Mangan sein. Besonders bevorzugt werden Magnesium/Aluminiummischoxide. Die Mischoxide können oberflächlich modifiziert sein mit einem oder mehreren der bereits genannten Co-Katalysatoren, insbesondere mit den Hydroxiden und/oder Alkoxiden der Alkali- und/oder Erdalkalimetalle. Derartige Mischmetalloxide und deren Modifizierungsmöglichkeiten werden beispielsweise in der deutschen Offenlegungsschrift **DE-A- 44 46 064** beschrieben.

Besonders bevorzugt wird als Co-Katalysator Magnesiumoxid.

Sofern die ggf. modifizierten Hydrotalcite alleinige Katalysatoren sind, werden sie üblicherweise in Mengen von 0,1 bis 5, vorzugsweise in Mengen von 0,5 bis 1,5 Gew.% - bezogen auf Ausgangsverbindungen (Verbindungen mit aktivem Wasserstoff bzw. Carbonsäureester und Alkylenoxid) eingesetzt.

Sofern die ggf. modifizierten Hydrotalcite zusammen mit den ausgewählten Co-Katalysatoren eingesetzt werden, werden sie üblicherweise in Mengen von 0,05 bis 2,5 und insbesondere von 0,1 bis 0,5 Gew.% - bezogen auf die Ausgangsverbindungen - eingesetzt. Die Co-Katalysatoren können in Mengen von 0,05 bis 5, vorzugsweise in Mengen von 0,1 bis 0,5 und insbesondere in Mengen von 0,1 bis 0,3 Gew.% - bezogen auf die Ausgangsverbindungen - eingesetzt werden.

Aufgrund des synergistischen Effektes in der Aktivität als Katalysator zwischen ggf. modifiziertem Hydrotalcit und den Co-Katalysatoren ist es im Sinne der vorliegenden Erfindung sogar möglich, bei einer Gesamtmenge an Hydrotalcit und Co-Katalysatoren unter 0,5 Gew.%, vorzugsweise bei Mengen zwischen 0,1 und 0,4 und insbesondere zwischen 0,2 und 0,3 Gew.% - bezogen auf die Ausgangsverbindungen - sehr gute Ergebnisse zu erzielen.

Das Verhältnis zwischen ggf. modifiziertem Hydrotalcit als Katalysator und Co-Katalysatoren kann in weiten Bereichen schwanken, vorzugsweise liegt das Gewichtsverhältnis zwischen 5 : 1 bis 1 : 5, insbesondere zwischen 3 : 1 und 1 : 3 und insbesondere bevorzugt zwischen 2 : 1 und 1 : 2.

Die Umsetzung der Verbindungen mit aktiven Wasserstoffatomen bzw. der Carbonsäureester mit den Alkylenoxiden kann in an sich bekannter Weise bei Temperaturen von 120 bis 200 °C, vorzugsweise 150 bis 180 °C und Drücken von 1 bis 5 bar durchgeführt werden. Die Menge des anzulagernden Alkylenoxids ist dabei unkritisch und kann beispielsweise 1 bis 100, vorzugsweise 2 bis 50 und insbesondere 2 bis 20 Mol Alkylenoxid pro Mol H-aktiver Verbindung bzw. Carbonsäureester betragen.

Als Alkylenoxide können Ethylen-, Propylen- und/oder Butylenoxid eingesetzt werden, vorzugsweise Ethylenoxid.

Erfindungswesentlich ist nun, daß im Anschluß an die eigentliche Alkoxylierung die eingesetzten Katalysatoren, nämlich der ggf. modifizierte Hydrotalcit alleine oder in Mischung mit den ausgewählten Co-Katalysatoren, in der erhaltenen Reaktionsmischung insgesamt möglichst vollständig zersetzt werden durch die Zugabe an Säuren.

Als Säuren können sowohl anorganische als auch organische Säuren zugesetzt werden. Als anorganische Säuren eignen sich insbesondere die Mineralsäuren wie Schwefelsäure, Salzsäure und/oder Phosphorsäure. Als organische Säuren können sowohl die sogenannten Fettsäuren mit 6 bis 22 Kohlenstoffatomen als auch niedere Carbonsäuren mit 1 bis 4 Kohlenstoffatomen (gezählt werden nur die C-Atome des Kohlenwasserstoffgerüstes, nicht die C-Atome der Carboxylgruppen), die ggf. zusätzlich mit Hydroxylgruppen modifiziert sein können, eingesetzt. Bevorzugt werden als organische Säuren die niederen Carbonsäuren wie Milchsäure, Essigsäure und/oder Citronensäure.

Die Säuren werden als wäßrige Lösungen, vorzugsweise als 10 bis 90 Gew.%ige Lösungen zugegeben.

Die Säuren werden erfindungsgemäß mindestens in äquimolaren Mengen - bezogen auf Hydrotalcit und ggf. vorhandenen Co-Katalysatoren - eingesetzt. Die konkrete Zugabemenge an Säuren ist natürlich abhängig von der Säurestärke. In der Regel empfehlen sich molare Mengen an Säuren zu Hydrotalcit und ggf. Co-Katalysatoren von 1 : 1 bis 4 : 1.

Die Zugabe der Säuren bei Temperaturen oberhalb des Schmelzpunktes der alkoxylierten nichtionischen Tenside erfolgt bei Temperaturen zwischen 70 und 95 °C. Praktisch wird dies am besten so durchgeführt, dass man nach der Alkoxylierung die erhaltene Reaktionsmischung bei diesen Temperaturen hält und die Säure zugibt. Durch die Säurezugabe wird erreicht, daß der Hydrotalcit sich zersetzt. Die Zersetzungsprodukte des Hydrotalcites erweisen sich als wasserlöslich und führen nicht zu Austrübungen oder Sedimentationen der alkoxylierten nichtionischen Tenside. Demnach gelingt es, mit dem erfindungsgemäßen Verfahren auf die aufwendige Filtration des ggf. modifizierten Hydrotalcits zu verzichten. Falls gewünscht kann sich natürlich der erfindungsgemäßen Säurebehandlung eine Aufarbeitung der wasserlöslichen Zersetzungsprodukte des Hydrotalcits anschließen, beispielsweise durch eine Abtrennung der wässrigen Phase von der organischen Phase.

Die nach den erfindungsgemäßen Verfahren erhaltenen alkoxylierten nichtionischen Tenside sind ohne weitere Filtration einsetzbar. Auch nach längerer Lagerung treten keine Ausfällungen oder Sedimentationen auf Sie eignen sich daher als nichtionisches Tensid zur Herstellung von Wasch-, Spül und Reinigungsmitteln und zur Herstellung von reinigender Kosmetika, insbesondere von flüssigen Produkten wie flüssigen Textilwaschmitteln, Haarshampoos und dergleichen.

### Beispiele

### Beispiel 1

288,6 g (entsprechend 1,35 Mol) eines Methyllaurats wurden zusammen mit 5,0 g (entsprechend 0,5 Gew.% - bezogen auf Ausgangsverbindungen) calciniertem Hydrotalcit in einem Druckbehälter vorgelegt. Der Behälter wurde 30 Minuten bei 100 °C evakuiert und anschließend mit Stickstoff belüftet. Bei max. 180 °C und max. 5 bar Druck erfolgte portionsweise die Zudosierung von 711,4 g (entsprechend 12 Mol) Ethylenoxid. Die Reaktionszeit betrug 150 Minuten. Nach Beendigung der Alkoxylierung wurde eine Stunde bei 120 °C nachreagiert und nochmals 30 Minuten bei 120 °C die Apparatur evakuiert.

Zu dieser Reaktionsmischung wurden bei 90 °C unter Rühren 100 g einer 10 Gew.%igen Zitronensäure zugegeben.

Der calcinierte Hydrotalcit war nach 10-minütigem Rühren aufgelöst. Es wurde eine in der Schmelze klare Reaktionsmischung erhalten.

### Beispiel 2

Eine Mischung aus 65,3 g (entsprechend 0,41 Mol) eines Caprylsäuremethylesters und 261,3 g (entsprechend 1,12 Mol) eines Methylesters aus Palmkernöl (Fettsäurenkette mit 12 bis 18 Kohlenstoffatomen wurden mit 1,0 g (entsprechend 0,1 Gew.% bezogen auf Ausgangsverbindungen) calciniertem Hydrotalcit und 2,0 g Magnesiumoxid (entsprechend 0,2 Gew.% - bezogen auf Ausgangsverbindungen) in einem Druckbehälter vorgelegt. Der Behälter wurde 30 Minuten bei 100 °C evakuiert und anschließend mit Stickstoff belüftet. Bei max. 180 °C und max. 5 bar Druck erfolgte portionsweise die Zudosierung von 673,4 g (entsprechend 10 Mol) Ethylenoxid. Die Reaktionszeit betrug 160 Minuten. Nach Beendigung der Alkoxylierung wurde eine Stunde bei 120 °C nachreagiert und nochmals 30 Minuten bei 120 °C die Apparatur evakuiert.

Zu dieser Reaktionsmischung wurde unter Rühren bei 90 °C 68,4 g einer 20 Gew.%igen Essigsäure zugegeben. Innerhalb von drei Minuten war die Katalysatormischung aus Hydrotalcit und Magnesiumoxid vollständig aufgelöst.

## Patentansprüche

1. Verfahren zur Herstellung alkoxylierter nichtionischer Tenside durch Umsetzung von Carbonsäuren mit 6 bis 22 Kohlenstoffatomen oder Carbonsäureestern mit Alkylenoxiden in Gegenwart von ggf. modifiziertem Hydrotalcit als Katalysator und ggf. von Co-Katalysatoren, **dadurch gekennzeichnet, dass** nach der Alkoxylierung Säuren in Form wässriger Lösungen im Verhältnis zu Hydrotalcit und ggf. Co-Katalysatoren in molaren Mengen von 1 : 1 bis 10 : 1 bei Temperaturen zwischen 70 und 95 °C zugegeben werden.

2. Verfahren nach Anspruch 1, **dadurch gekennzeichnet, dass** die Säuren im Verhältnis zum Hydrotalcit und ggf. Co-Katalysatoren in molaren Mengen von 1 : 1 bis 4 : 1 zugegeben werden.

3. Verfahren nach Anspruch 1, **dadurch gekennzeichnet, dass** organische Säuren mit 1 bis 4 Kohlenstoffatomen zugegeben werden.

4. Verfahren nach einem der Ansprüche 1 bis 3, **dadurch gekennzeichnet, dass** die Säure als 10 bis 90 Gew.-%ige Lösung zugegeben werden.

5. Verfahren nach einem der Ansprüche 1 bis 4, **dadurch gekennzeichnet, dass** als Carbonsäureester Methylester von Carbonsäuren mit 6 bis 22 Kohlenstoffatomen eingesetzt werden.

6. Verfahren nach einem der Ansprüche 1 bis 5, **dadurch gekennzeichnet, dass** Co-Katalysatoren eingesetzt werden, die ausgewählt sind aus der Gruppe, die gebildet wird von Hydroxiden, Oxiden und/oder Alkoxiden von Alkali- und/oder Erdalkalimetallen sowie von Alkali- und/oder Erdalkalisalzen, Zinnsalzen und von Mischmetalloxiden.

## Claims

1. A process for the production of alkoxylated nonionic surfactants by reaction of C₆₋₂₂ carboxylic acids or carboxylic acid esters with alkylene oxides in the presence of optionally modified hydrotalcite as catalyst and optionally co-catalysts, **characterized in that**, after the alkoxylation, acids in the form of aqueous solutions are added in a molar ratio to hydrotalcite and co-catalysts (if any) of 1:1 to 10:1 at temperatures of 70 to 95°C.

2. A process as claimed in claim 1, **characterized in that** the acids are added in a molar ratio to hydrotalcite and co-catalysts (if any) of 1:1 to 4:1.

3. A process as claimed in claim 1, **characterized in that** organic acids containing 1 to 4 carbon atoms are added.

4. A process as claimed in any of claims 1 to 3, **characterized in that** the acids are added in the form of 10 to 90% by weight solutions.

5. A process as claimed in any of claims 1 to 4, **characterized in that** methyl esters of carboxylic acids containing 6 to 22 carbon atoms are used as the carboxylic acid esters.

6. A process as claimed in any of claims 1 to 5, **characterized in that** co-catalysts selected from the group consisting of hydroxides, oxides and/or alkoxides of alkali metals and/or alkaline earth metals and of alkali metal and/or alkaline earth metal salts, tin salts and of mixed metal oxides are used.

## Revendications

1. Procédé de fabrication d'agents tensioactifs non ioniques alcooxylés par transformation d'acides carboxyliques ayant 6 à 22 atomes de carbone ou d'esters d'acide carboxylique avec des oxydes d'alkylène, en présence d'hydrotalcite éventuellement modifié en tant que catalyseur, et éventuellement de co-catalyseurs,
**caractérisé en ce que**
après alcooxylation, des acides sous forme de solutions aqueuses proportionnellement à l'hydrotalcite et éventuellement des co-catalyseurs dans des quantités molaires de 1:1 à 10:1 sont ajoutés à des températures entre 70 et 95°C.

2. Procédé selon la revendication 1,
**caractérisé en ce que**
les acides sont ajoutés proportionnellement à l'hydrotalcite et les co-catalyseurs éventuels dans des quantités molaires de 1:1 à 4:1.

3. Procédé selon la revendication 1,
**caractérisé en ce que**
des acides organiques ayant 1 à 4 atomes de carbone sont ajoutés.

4. Procédé selon l'une des revendications 1 à 3,
**caractérisé en ce que**
les acides sont ajoutés en tant que solution à 10-90 % en poids.

5. Procédé selon l'une des revendications 1 à 4,
**caractérisé en ce que**
des esters méthyliques d'acides carboxyliques ayant 6 à 22 atomes de carbone sont utilisés comme esters d'acides carboxyliques.

6. Procédé selon l'une des revendications 1 à 5,
**caractérisé en ce que**
l'on utilise des co-catalyseurs qui sont choisis dans le groupe composé d'hydroxydes, d'oxydes et/ou d'alcoxydes de métaux alcalins et/ou alcalinoterreux ainsi que de sels d'alcalins et/ou d'alcalinoterreux, de sels d'étain et d'oxydes métalliques mixtes.
